# EUROPEAN PATENT APPLICATION

(11) **EP 2 050 448 A1**
(43) Date of publication of application: **22.04.2009**
(21) Application number: 07791902.5
(22) Date of filing: 03.08.2007
(51) Int. Cl.: A61K 31/4035, A61K 9/20, A61K 47/10, A61K 47/26, A61K 47/32, A61K 47/36, A61K 47/38, A61P 3/10

(54) **ORAL DISINTEGRATING TABLET HAVING MASKED BITTER TASTE AND METHOD FOR PRODUCTION THEREOF**

(30) Priority: 08.08.2006 JP 2006215146
(71) Applicant: Kissei Pharmaceutical Co., Ltd., Matsumoto-shi Nagano 399-8710 (JP)
(72) Inventor: MIMURA, Kazuki, Azumino-shi, Nagano 3998304 (JP); TAKEDA, Yasuhiro, Azumino-shi, Nagano 3998304 (JP); KANADA, Ken, Azumino-shi, Nagano 3998304 (JP)
(74) Representative: Hayes, Adrian Chetwynd
(86) International application number: PCT/JP2007/065228
(87) International publication number: WO 2008/018371

(57) **Abstract**

The present invention provides an orally disintegrating tablet containing mitiglinide calcium hydrate. The tablet has reduced bitterness and quickly disintegrates in the mouth, while exhibiting rapid dissolution in the digestive tract. The bitterness-masked orally disintegrating tablet comprises: (a) mitiglinide calcium hydrate; (b) microcrystalline cellulose; (c) at least one masking agent selected from the group consisting of aminoalkyl methacrylate copolymer E, polyvinylacetal diethylaminoacetate, an ethyl acrylate-methyl methacrylate copolymer, and ethyl cellulose; (d) a sugar or a sugar alcohol; and (e) at least one selected from corn starch and partially pregelatinized starch.

## Description

### Technical Field

The present invention relates to a bitterness-masked orally disintegrating tablet containing mitiglinide calcium hydrate, and a method for preparing such tablets.

### Background Art

Mitiglinide calcium hydrate (chemical name: (+)-Monocalcium bis[(2S,3a,7a-*cis*)-α-benzylhexahydro-y-oxo-2-isoindolinebutyrate]dihydrate) has an activity to improve postprandial hyperglycemia in type-2 diabetes mellitus. The mechanism of action involves binding to the sulfonylurea receptors of the pancreatic β cells to inhibit ATP-dependent K⁺ channel currents and thereby promoting insulin secretion (see Non-Patent Document 1, for example).

Mitiglinide calcium hydrate is commercially available as a tablet preparation, intended for oral administration in a single dose of 5 to 20 mg for adults, three times a day. Mitiglinide calcium hydrate is taken immediately before each meal, or more preferably within 5 minutes before meal, because absorption is slow and the efficacy attenuates in postprandial administration. In order to provide an insulin secretagogue capable of quickly exhibiting its action after administration, there have been developments of mitiglinide calcium hydrate-containing preparations that can rapidly dissolve in the digestive tract.

There have also been developments of solid preparations that can quickly disintegrate or dissolve in the mouth, in an effort to provide a dosage form readily administrable to the elderly, children, and patients having a problem with swallowing, or a dosage form that does not require water for administration.

WO2003/61650 discloses an orally disintegrating tablet containing (a) mitiglinide calcium hydrate, and (b) granules of co-spray dried lactose and starch (see Patent Document 1, for example). However, WO2003/61650 does not disclose anything about a bitterness-masked orally disintegrating tablet of mitiglinide calcium hydrate.

WO00/71117 discloses an immediate-release medicinal composition for oral use, containing mitiglinide calcium hydrate as an active ingredient (see Patent Document 2, for example). However, the medicinal composition disclosed in WO00/71117 is an immediate-release tablet for the digestive tract such as the stomach, and the publication does not disclose anything about tablets that can quickly disintegrate in the mouth, nor does it disclose bitterness-masked orally disintegrating tablets.
Non-Patent literature 1: Ohnota H. et al., J. Pharmacol. Exp. Ther., 1994, vol. 269, p. 489-495
Patent literature 1: A pamphlet of International Publication 2003/61650
Patent literature 2: A pamphlet of International Publication 2000/71117
Patent literature 3: JP-A-4-235136
Patent literature 4: JP-A-2004-339071
Patent literature 5: A pamphlet of International Publication 2002/002083

### Disclosure of the Invention

The inventors of the present invention conducted studies on orally disintegrating tablets containing mitiglinide calcium hydrate. The studies found that mitiglinide calcium hydrate produces a strong bitter taste during administration. Because the orally disintegrating tablets are designed to quickly disintegrate in the mouth, the influence of bitterness becomes a big factor when the active ingredient has a bitter taste. It was also found that, because the mitiglinide calcium hydrate does not easily dissolve in water, simply disintegrating the tablet in the mouth is not sufficient to rapidly dissolve the compound in the digestive tract. Under these circumstances, the inventors of the present invention conducted studies to provide a mitiglinide calcium hydrate-containing orally disintegrating tablet having reduced bitterness and capable of rapidly dissolving in the digestive tract.

Various methods have been proposed to reduce bitterness, using, for example, a flavoring agent or a gel-forming anionic polymer (see Patent Document 3, for example), and a water-insoluble polymer (see Patent Document 4, for example). The inventors of the present invention applied these techniques to mitiglinide calcium hydrate. However, a sufficient masking effect could not be obtained with the addition of a flavoring agent or a gel-forming anionic polymer. Adding a water-insoluble substance reduced bitterness, but the dissolution of the drug was delayed in this case. A method is proposed in which the masking effect is provided by spray drying a mixture of a bitter drug and an insoluble polymer (see Patent Document 5, for example). However, this technique is not applicable to mitiglinide calcium hydrate, because of the strong water repellency of mitiglinide calcium hydrate.

The inventors of the present invention further conducted intensive studies on orally disintegrating tablets using a water-insoluble substance as a masking agent. As a result, it was found that an orally disintegrating tablet having considerably reduced bitterness and capable of rapidly dissolving in the digestive tract can be obtained when it includes a granulated material formed from mitiglinide calcium hydrate, microcrystalline cellulose, and a water-insoluble substance. The inventors of the present invention also found that an orally disintegrating tablet having an appropriate hardness and capable of quickly disintegrating in the mouth can be obtained when it is prepared from such a mitiglinide calcium hydrate-containing granulated material, a sugar or a sugar alcohol, and at least one selected from corn starch and partially pregelatinized starch. The present invention was accomplished based on these findings.

Specifically, the present invention provides:
[1] a bitterness-masked orally disintegrating tablet, comprising:
   (a) mitiglinide calcium hydrate as a bitter active ingredient;
   (b) microcrystalline cellulose;
   (c) at least one masking agent selected from the group consisting of aminoalkyl methacrylate copolymer E, polyvinylacetal diethylaminoacetate, an ethyl acrylate-methyl methacrylate copolymer, and ethyl cellulose;
   (d) a sugar or a sugar alcohol; and
   (e) at least one selected from corn starch and partially pregelatinized starch;
[2] a bitterness-masked orally disintegrating tablet, comprising:
   a granulated material including:
      (a) mitiglinide calcium hydrate as a bitter active ingredient;
      (b) microcrystalline cellulose; and
      (c) at least one masking agent selected from the group consisting of aminoalkyl methacrylate copolymer E, polyvinylacetal diethylaminoacetate, an ethyl acrylate-methyl methacrylate copolymer, and ethyl cellulose;
      (d) a sugar or a sugar alcohol; and
      (e) at least one selected from corn starch and partially pregelatinized starch;
[3] an orally disintegrating tablet according to [1] or [2], wherein the sugar or the sugar alcohol is lactose or D-mannitol;
[4] an orally disintegrating tablet according to [1] or [2], wherein the sugar or the sugar alcohol is D-mannitol;
[5] an orally disintegrating tablet according to [1] or [2], wherein the masking agent is at least one selected from aminoalkyl methacrylate copolymer E and polyvinylacetal diethylaminoacetate;
[6] an orally disintegrating tablet according to [2], wherein the granulated material is obtained by granulating a mixture of mitiglinide calcium hydrate and microcrystalline cellulose while spraying at least one masking agent selected from the group consisting of aminoalkyl methacrylate copolymer E, polyvinylacetal diethylaminoacetate, an ethyl acrylate-methyl methacrylate copolymer, and ethyl cellulose;
[7] an orally disintegrating tablet according to [2],
   wherein the granulated material has an average particle diameter of 60 to 150 µm;
[8] a bitterness-masking particle for orally disintegrating tablets,
   the particle including:
   (a) mitiglinide calcium hydrate;
   (b) microcrystalline cellulose; and
   (c) at least one masking agent selected from aminoalkyl methacrylate copolymer E, polyvinylacetal diethylaminoacetate, an ethyl acrylate-methyl methacrylate copolymer, and ethyl cellulose;
[9] a bitterness-masking particle according to [8], wherein the bitterness-masking particle has an average particle diameter of 60 to 150 µm;
[10] a method for preparing a bitterness-masked orally disintegrating tablet,
   the method comprising the steps of:
   (1) granulating a mixture of mitiglinide calcium hydrate and microcrystalline cellulose while spraying at least one masking agent selected from the group consisting of aminoalkyl methacrylate copolymer E, polyvinylacetal diethylaminoacetate, an ethyl acrylate-methyl methacrylate copolymer, and ethyl cellulose; and
   (2) compression-molding the granulated material obtained in the granulating step, after mixing the granulated material with a sugar or a sugar alcohol, and at least one selected from corn starch and partially pregelatinized starch; and
[11] a method according to [10], wherein the granulation in the granulating step is performed by a high shear granulating method.

Preferably, the masking agent used for an orally disintegrating tablet of the present invention is water-insoluble, and delays the dissolution of the drug in the mouth. Examples of such masking agents include a stomach-soluble polymer, a water-insoluble cellulose ether, and a water-insoluble acrylic polymer. Examples of the stomach-soluble polymer include methyl methacrylate-butyl methacrylate-dimethylaminoethyl methacrylate copolymers such as aminoalkyl methacrylate copolymer E (For example, Eudragit EPO, Roehm Pharma Gmbh; Eudragit E100, Roehm Pharma Gmbh), and stomach-soluble polyvinyl derivatives such as polyvinylacetal diethylaminoacetate (for example, AEA, Sankyo). Examples of the water-insoluble cellulose ether include ethyl celluloses (for example, Ethocel STD10FP, Dow Chemical Company), and aqueous dispersions of ethyl cellulose (for example, Aquacoat, FMC). Examples of the water-insoluble acrylic polymer include dispersion liquids of ethyl acrylate-methylmethacrylate copolymer (for example, Eudragit NE30D, Roehm Pharma Gmbh). Among these masking agents, the stomach-soluble polymer, capable of rapidly dissolving in the stomach, is preferable in terms of masking effect and solubility. In the same respect, aminoalkyl methacrylate copolymer E and polyvinylacetal diethylaminoacetate are most preferable. As required, these masking agents may be used in a combination of two or more.

When a water-insoluble substance is added as a masking agent, the property of the tablet to disintegrate and disperse suffers, which, in turn, lowers the dissolution property of the tablet, or the drug dissolution from the drug-containing particles of the disintegrating tablet. It is therefore required that the water-insoluble substance be added in such amounts sufficient to reduce bitterness in the mouth but not detrimental to the rapid dissolution of the drug in the digestive tract. In an orally disintegrating tablet of the present invention, the content of the masking agent, though it depends on the type of masking agent, is generally about 1 to about 100 parts by weight, preferably about 5 to about 50 parts by weight, and more preferably about 10 to about 50 parts by weight, with respect to 100 parts by weight of mitiglinide calcium hydrate.

As mentioned above, the mitiglinide calcium hydrate-containing preparation is administered immediately before meal, and preferably, rapidly dissolves upon administration to improve postprandial hyperglycemia. It is therefore desirable in an orally disintegrating tablet of the present invention that the tablet rapidly dissolves in the digestive tract, particularly in the stomach, after having disintegrated in the mouth. The mitiglinide calcium hydrate has a calcium salt of carboxylic acid as a functional group within the molecule, making it soluble in an alkaline pH range, and insoluble toward the neutral to acidic pH. It is therefore preferable that an orally disintegrating tablet of the present invention rapidly dissolve in the stomach and water.

In an orally disintegrating tablet of the present invention, microcrystalline cellulose, after the disintegrating tablet is disintegrated in the mouth, improves the wetting and dispersibility of the mitiglinide calcium hydrate in the digestive tract, and particularly in the stomach, to thereby improve the dissolution property of the mitiglinide calcium hydrate. Examples of the microcrystalline cellulose used in an orally disintegrating tablet of the present invention include Ceolus PH-101, PH-102, PH-301, PH-302, F-20, and KG-802 (Asahi Kasei Chemicals Corporation), which may be used in a combination of two or more. The content of microcrystalline cellulose in an orally disintegrating tablet of the present invention is generally about 10 to about 500 parts by weight, and preferably about 30 to about 300 parts by weight, with respect to 100 parts by weight of mitiglinide calcium hydrate.

The sugar or sugar alcohol used in an orally disintegrating tablet of the present invention is preferably highly water-soluble, and exhibits low moldability. Examples of such sugars and sugar alcohols include: sugars such as lactose, glucose, sucrose, and fructose; and sugar alcohols such as D-mannitol, erythritol, and xylitol. Lactose and D-mannitol are preferable for their pleasant sweet taste exhibited during administration. Of these, D-mannitol is particularly preferable for its ability to provide a pleasant, cooling sensation, while having an appropriate hardness and facilitating the tablet to quickly disintegrate.
Examples of the lactose used in an orally disintegrating tablet of the present invention include Tablettose 70, Tablettose 80, Tablettose 100 (Meggle), Pharmatose 100M, Pharmatose 200M, Impalpable (DMV), and FAST-FLO (Formost). Among these examples, the direct tableting lactose Tablettose 70, Tablettose 80, Tablettose 100 (Meggle), FAST-FLO (Formost), and Pharmatose 100M (DMV) are preferable. Examples of the D-mannitol used in an orally disintegrating tablet of the present invention include Mannit P (Towa Kasei Kogyo), PEARLITOL 25C, PEARLITOL 50C, PEARLITOL 100SD, PEARLITOL 200SD, and PEARLITOL 400DC (Roquette).
As required, these sugars or sugar alcohols may be used in a combination of two or more. Further, the sugar and sugar alcohol may be used in combination.

In an orally disintegrating tablet of the present invention, the content of sugar or sugar alcohol is about 10 to about 95 parts by weight, preferably about 30 to about 90 parts by weight, and more preferably about 40 to about 90 parts by weight, with respect to 100 parts by weight of the tablet.

In an orally disintegrating tablet of the present invention, corn starch and partially pregelatinized starch are used to help the tablet quickly disintegrate in the mouth, and to give an appropriate hardness to the tablet. Examples of the partially pregelatinized starch used in an orally disintegrating tablet of the present invention include Starch 1500 (Colorcon Japan, cold-water solubles: 10 to 20 weight%), PCS (Asahi Kasei Chemicals Corporation, cold-water solubles: less than 10 weight%), LYCATAB C (Roquette, cold-water solubles: less than 10 weight%), and Fibose (Nippon Starch Chemical Co., Ltd.). Among these, Starch 1500 (Colorcon Japan, cold-water solubles: 10 to 20 weight%) is preferable.

In an orally disintegrating tablet of the present invention, the content of corn starch is about 2 to about 40 parts by weight, and preferably about 5 to about 30 parts by weight, with respect to the total weight of the preparation. In an orally disintegrating tablet of the present invention, the content of partially pregelatinized starch is about 0.5 to about 10 parts by weight, and preferably about 1 to about 5 parts by weight, with respect to 100 parts by weight of the tablet.

The content of mitiglinide calcium hydrate in an orally disintegrating tablet of the present invention is not particularly limited to, but generally about 1 to about 20 parts by weight, and preferably about 2 to about 10 parts by weight, with respect to 100 parts by weight of the tablet.

An orally disintegrating tablet of the present invention may include appropriate amounts of a variety of additives used for production of preparations, provided that they do not interfere with the effects of the present invention. Examples of such additives include fillers, binders, lubricants, sweeteners, acidulants, foaming agents, flavoring agents, and colorants.

Examples of the fillers include rice starch, potato starch, magnesium aluminometasilicate, anhydrous calcium phosphate, precipitated calcium carbonate, calcium silicate, calcium lactate, and ethyl cellulose. Examples of the binders include hydroxypropyl cellulose, hydroxypropylmethyl cellulose, polyvinylpyrrolidone, dextrin, methyl cellulose, polyvinyl alcohol, sodium alginate, aminoalkyl methacrylate copolymers, and polyethylene glycols. Examples of the lubricants include magnesium stearate, calcium stearate, talc, light anhydrous silicic acid, sucrose fatty acid esters, and sodium stearyl fumarate. Examples of the sweeteners include Aspartame^{®}, saccharine sodium, dipotassium glycyrrhizinate, stevia, thaumatin, acesulfame K, and sucralose. Examples of the acidulants include citric acid, tartaric acid, malic acid, and ascorbic acid. Examples of the foaming agents include sodium bicarbonate, sodium carbonate, and calcium carbonate. Examples of the flavoring agents include L-aspartic acid, sodium chloride, magnesium chloride, sodium citrate, calcium citrate, L-sodium glutamate, and sodium bicarbonate. Examples of the other flavoring agents include orange oil, lemon oil, menthol, and various kinds of flavoring agent powders. Examples of the colorants include: food dyes such as food yellow 5, food red 2, and food blue 2; yellow ferric oxide; red ferric oxide; and caramel dyes.

The following describes a method for preparing an orally disintegrating tablet of the present invention.

A method for preparing an orally disintegrating tablet of the present invention includes the steps of:
(1) granulating a mixture of mitiglinide calcium hydrate and microcrystalline cellulose while spraying at least one kind of masking agent selected from the group consisting of aminoalkyl methacrylate copolymer E, polyvinylacetal diethylaminoacetate, an ethyl acrylate-methyl methacrylate copolymer, and ethyl cellulose; and
(2) compression-molding the granulated material after mixing it with a sugar or a sugar alcohol, and at least one selected from corn starch and partially pregelatinized starch.
   In a method for preparing an orally disintegrating tablet of the present invention, the granulated material includes microcrystalline cellulose and a water-insoluble polymer to mask the bitterness of the mitiglinide calcium hydrate and provide rapid drug dissolution. Further, by the compression molding of the granulated material mixed with a sugar or a sugar alcohol, and at least one selected from corn starch and partially pregelatinized starch, the tablet is sufficiently hard and quickly disintegrates in the mouth.

The following specifically describes each step of a preparing method of the present invention.

### (Granulating Step)

Mitiglinide calcium hydrate contained in an orally disintegrating tablet of the present invention has low fluidity, in addition to being very adherent and water-repellent. This makes it difficult to directly granulate the mitiglinide calcium hydrate using a solution or suspension of the masking agent as a liquid binder. Further, when a mixture of mitiglinide calcium hydrate and an excipient such as D-mannitol is used to prepare an orally disintegrating tablet by compression molding after granulating the mixture using the masking agent as a liquid binder, the resulting tablet suffers from low dissolution, though it can mask the bitterness. As a result of intensive studies, the inventors of the present invention found that the bitterness-masking effect and rapid drug dissolution can be realized at the same time, when a mixture of mitiglinide calcium hydrate and microcrystalline cellulose is granulated using the masking agent as a liquid binder.

Regarding the granulating step, use of a fluidized bed granulating method or a tumbling fluidized bed granulating method causes a problem in that, owning to the high adherence of the mitiglinide calcium hydrate, the drug in the flowing air adheres to the upper part inside the granulating apparatus, causing the drug to granulate separately from the microcrystalline cellulose. The resulting granulated material is therefore bulky and friable, which causes the granulated material to break during the compression molding. That is, a sufficient bitterness-masking effect cannot be obtained in tablets prepared by a fluidized bed granulating method or a tumbling fluidized bed granulating method. When a spray drying granulating method is used, the high water-repellency of the mitiglinide calcium hydrate prevents formation of a spray solution of mitiglinide calcium hydrate and masking agent. In a preparing method of the present invention, the granulating step is preferably performed by making a mixture of mitiglinide calcium hydrate and microcrystalline cellulose using a high shear granulating method, and granulating the mixture while spraying a solution or dispersion of the masking agent as a liquid binder.

The solvent used to dissolve or suspend the masking agent is not particularly limited to, but includes alcohols such as ethanol and methanol; methylene chloride; toluene; methyl ethyl ketone; water; and mixtures of these. Ethanol and water are preferable. The ethyl acrylate-methyl methacrylate copolymer (for example, Eudragit NE 30D, Roehm Pharma Gmbh) and ethyl cellulose (for example, Aquacoat, FMC) are commercially available in the form of an aqueous dispersion, and may be used by being diluted with water, as required. The aminoalkyl methacrylate copolymer E, which is water-insoluble, may be used as an aqueous solution by being dissolved in acidic water (pH of 5 or less), or an aqueous dispersion by being mixed, in any proportion, with at least one kind of plasticizer selected from sodium lauryl sulfate, stearic acid, triethyl citrate, diethyl sebacate, and dibutyl sebacate.

The liquid binder of masking agent may additionally include additives used for production of preparations, provided that it is not detrimental to the bitterness-masking effect and dissolution properties. Examples of such additives include: binders such as hydroxypropyl cellulose, hydroxypropylmethyl cellulose, polyethylene glycol, and polyvinylpyrrolidone; colorants such as red ferric oxide, yellow ferric oxide, food dyes, and caramel dyes; and surfactants such as sodium laurate, sucrose fatty acid esters, diethyl sebacate, cetanol, Polysorbate 80, and Macrogol 400.

In an orally disintegrating tablet of the present invention, the average particle diameter of the granulated material is preferably about 60 to about 150 µm, and more preferably about 60 to about 120 µm. When the average particle diameter of the granulated material is below 60 µm, a sufficient bitterness-masking effect cannot be obtained. Above 150 µm, rapid drug dissolution suffers. In the present invention, the "average particle diameter" means a 50% particle diameter (weight-based median size). The 50% particle diameter can be measured with a particle distribution measuring sifter (for example, sonic sifter L-3PS, Seishin Enterprise Co., Ltd.).

In a preparing method of the present invention, the granulating step is preferably performed by granulating a mixture of mitiglinide calcium hydrate and microcrystalline cellulose while spraying a solution or dispersion of the masking agent through a spray nozzle, after thoroughly mixing the mitiglinide calcium hydrate and microcrystalline cellulose using a high shear granulating method.

Generally, in a high shear granulating method, various factors are known to influence ease of granulation. Some of the examples include the method of adding the liquid binder, the concentration of the liquid binder, the amount of liquid binder added, granulation time, the number of blade rotations, and the number of cross screw rotations.
The method of adding the liquid binder, the amount of liquid binder added, granulation time, and the number of blade rotations and cross screw rotations in a high shear granulator are particularly important in a preparing method of the present invention. The liquid binder is added preferably by a spray method, because the proportion of coarse particles increases in a falling-drop method. The rotation speed of the blade and the cross screw in the high shear granulator is preferably about 15 to about 600 rpm for the blade, and preferably about 180 to about 3,600 rpm for the cross screw, though it depends on the manufacturing scale. Regarding the amount of liquid binder added, the average particle diameter of the granulated material generally increases as the amount of liquid binder is increased, and decreases when the granulation time becomes excessively long. In a preparing method of the present invention, the amount of liquid binder added and the granulation time are appropriately adjusted according to such factors as the manufacturing scale, the type of masking agent, and the type of solvent used to dissolve or suspend the masking agent, so as to produce a granulated material having an average particle diameter of about 60 to about 150 µm.

The granulated material may be further coated with a masking agent to such an extent that the dissolution of the drug from the preparation is not overly delayed. The coating step does not particularly limit the method of production, and methods such as a fluidized bed coating method, a tumbling fluidized bed coating method, a Wurster coating method, and a melt coating method may be used.
The coating step can further improve the bitterness-masking effect.

### (Mixing Step)

In a preparing method of the present invention, the mixing step proceeds by mixing the mitiglinide calcium hydrate-containing granulated material, prepared in the granulating step, with (a) a sugar or a sugar alcohol, and (b) at least one selected from corn starch and partially pregelatinized starch.

When the sugar used in a preparing method of the present invention is for direct tableting, mixing and compression molding of (a) the mitiglinide calcium hydrate-containing granulated material, (b) sugar, and (c) corn starch can form a tablet of appropriate hardness that can quickly disintegrate in the mouth.

When the sugar alcohol used makes it difficult to perform the direct mixing and compression molding with the mitiglinide calcium hydrate-containing granulated material, it is desirable that the sugar alcohol be granulated beforehand to improve fluidity and ease of feeding. For example, when D-mannitol is used as a sugar alcohol, a partially pregelatinized starch, and particularly a partially pregelatinized starch having about 10 to about 20 weight% of cold-water solubles, and specifically Starch 1500 (Colorcon Japan, cold-water solubles: 10 to 20 weight%) are preferably used as a granulation binder, because they reduce the incidence of tableting failures and provide a tablet of appropriate hardness that can quickly disintegrate in the mouth.

The granulation of the sugar alcohol can be performed, for example, by granulating a mixture of (a) a sugar alcohol and (b) corn starch using a partially pregelatinized starch as a binder. In the granulation of the sugar alcohol, (1) a granulated material including (a) a sugar alcohol, (b) corn starch, and (c) partially pregelatinized starch may be prepared first and a remaining part of corn starch may be added and mixed thereafter, or alternatively (2) the entire amount of corn starch may be added and mixed after preparing a granulated material including (a) a sugar alcohol and (b) partially pregelatinized starch.

The granulation of the sugar alcohol may be performed by common wet granulating methods, such as, for example, a high shear granulating method, a fluidized bed granulating method, a tumbling fluidized bed granulating method, and an extrusion granulating method. Preferably, a high shear granulating method and a fluidized bed granulating method are used.

After preparing a mixture of the mitiglinide calcium hydrate-containing granulated material with (a) a sugar or a sugar alcohol, and (b) at least one selected from corn starch and partially pregelatinized starch, additives such as lubricants, foaming agents, sweeteners, flavoring agents, fluidizers and flavoring agents may be added as required.

In an orally disintegrating tablet of the present invention, the compression molding can be performed using, for example, a single punch tableting machine or a rotary tableting machine. The punch pressure is generally 1 to 60 kN/cm², and preferably 3 to 30 kN/cm².

An orally disintegrating tablet of the present invention, produced as above, has an appropriate hardness, and can quickly disintegrate in the mouth with its bitterness masked. Further, an orally disintegrating tablet of the present invention exhibits rapid drug disolution in the digestive tract after having disintegrated in the mouth. Further, an orally disintegrating tablet prepared by a preparing method of the present invention is suited for industrial production, because it is free of tableting failures during the compression molding.

To provide a sufficient bitterness-masking effect, an orally disintegrating tablet of the present invention preferably has an average score of less than 2.0 in the bitterness test described below.

The disintegration time of an orally disintegrating tablet of the present invention in the mouth is generally within 60 seconds, preferably within 40 seconds, and more preferably within 30 seconds, though it depends on the size or thickness of the tablet. The hardness of an orally disintegrating tablet of the present invention is generally 30 N or more, and preferably 50 N or more.

An orally disintegrating tablet of the present invention preferably has good dissolution properties in the first fluid (pH of about 1.2), equivalent of stomach pH, and in purified water. Specifically, the drug dissolution rate of an orally disintegrating tablet of the present invention is preferably 85% or more after 15 minutes when a dissolution test is conducted at a rotation speed of 50 rpm using the first fluid (pH of about 1.2) as a test fluid according to method 2 (paddle method) in the dissolution test of the Japanese Pharmacopoeia, Fourteenth Edition, and more preferably 85% or more in both the first fluid (pH of about 1.2) and purified water as test fluids after 15 minutes when a dissolution test is conducted at a rotation speed of 50 rpm according to method 2 (paddle method) in the dissolution test of the Japanese Pharmacopoeia, Fourteenth Edition.

A mitiglinide calcium hydrate-containing orally disintegrating tablet of the present invention is generally taken in a mitiglinide calcium hydrate dose of 5 to 20 mg for adults, three times a day immediately before each meal, and preferably 5 minutes before each meal.

### Advantageous effects of the Invention

An orally disintegrating tablet of the present invention masks the bitterness attributed to the mitiglinide calcium hydrate, and quickly disintegrates in the mouth, making it easier for patients to take. Further, because an orally disintegrating tablet of the present invention rapidly dissolves in the digestive tract after having disintegrated in the mouth, it can effectively suppress postprandial hyperglycemia. Further, the bitterness-reduced, orally disintegrating tablet of the present invention is easy to handle because it is sufficiently hard to withstand damages encountered during the course of distribution.

### Best Mode for Carrying Out the Invention

The following describes the present invention in more detail based on Examples, Comparative Examples, and Test Examples below. Note, however, that the invention is not limited in any ways by the following descriptions.

### Examples

### Measurement of Particle Distribution

A particle distribution was measured to determine a 50% particle diameter (weight-based median size) by sifting, using a sonic sifter (model L-3PS, Seishin Enterprise Co., Ltd.).

### Test Example 1

### Bitterness Test

Each tablet prepared in Examples 1 to 5 and Comparative Examples 1 to 6 was put in the mouth of five healthy males. The tablet was gently rolled on the tongue until it disintegrated, and furthermore was kept in the mouth for 30 seconds. Then, bitterness was scored according to Table 1, and the average was taken.

**[Table 1]**

| | |
|---|---|
| 0 | None |
| 1 | Almost none |
| 2 | Slightly bitter |
| 3 | Bitter |
| 4 | Very bitter |
| 5 | Extremely bitter |

### Test Example 2

### Dissolution Test

Each tablet prepared in Examples 1 to 5 and Comparative Examples 1 to 6 was conducted a dissolution test to determine dissolution rate after 15 minutes. The test was performed at a paddle rotation speed of 50 rounds per minute (rpm) using 900 mL of purified water or 900 mL of the first fluid as test fluids, according to method 2 (paddle method) in the dissolution test of the Japanese Pharmacopoeia, Fourteenth Edition.

### Test Example 3

### Oral Disintegration Test

Each tablet prepared in Examples 1 to 5 was put in the mouth of five healthy males. The tablet was gently rolled on the tongue until it disintegrated, and the time required to disintegrate the tablet was measured and averaged.

### Test Example 4

### Hardness Test

The hardness of each tablet prepared in Examples 1 to 5 was measured using a hardness meter (TS-75N, Okada Seiko Co., Ltd.).

### Example 1

| | |
|---|---|
| Mitiglinide calcium hydrate: | 10.0 mg |
| Microcrystalline cellulose: | 25.0 mg |
| Polyvinylacetal diethylaminoacetate: | 1.0 mg |
| D-mannitol: | 76.5 mg |
| Partially pregelatinized starch: | 2.5 mg |
| Corn starch: | 30.0 mg |
| Red ferric oxide: | 0.005 mg |
| Aspartame: | 2.0 mg |
| Calcium stearate: | 2.0 mg |
| Light anhydrous silicic acid: | 1.0 mg |
| Total: | 150.0 mg/tablet |

1,000 g of mitiglinide calcium hydrate and 2,500 g of microcrystalline cellulose (Ceolus PH-101, Asahi Kasei Chemicals Corporation) were mixed using a high shear granulator (FM-VG-25, Powrex Corporation). For granulation, a solution prepared by dissolving 100 g of polyvinylacetal diethylaminoacetate (AEA, Sankyo) in 1,150 g of 90 weight% ethanol was sprayed onto the mixture using a two-fluid spray nozzle at a feed rate of 250 g/min. Here, the mixture was granulated for a total of 15 minutes at a blade rotation speed of 250 rpm and a cross screw rotation speed of 2,000 rpm. The wet granulated material was dried with a tray drier (DSB80HPT, Seiwa Rikou), and sized using a mill with a screen having ∅ 0.55 mm opening (ND-30S, Okada Seiko Co., Ltd.). The resulting sized granulated material containing mitiglinide calcium hydrate (a-1) had a 50% particle diameter of 75.5 µm.
Separately, 1,200 g of D-mannitol (Mannite P, Towa Kasei Kogyo), and 40 g of corn starch (Nihon Shokuhin Kako Co., Ltd.) were charged into a fluidized bed granulator (LAB-1, Powrex Corporation). Then, a dispersion liquid, prepared by dispersing 40 g of partially pregelatinized starch (Starch 1500, Colorcon Japan) and 0.08 g of red ferric oxide in 360 g of purified water, was sprayed through a spray nozzle to granulate. The resulting granulated material was sized using a mill with a screen having ∅ 1.5 mm opening (P-02S, Dalton Co., Ltd.) to obtain a fluidized bed granulated material (b-1).
504 g of sized granulated material containing mitiglinide calcium hydrate (a-1), 1,141 g of fluidized bed granulated material (b-1), 385 g of corn starch, and 28 g of aspartame (Ajinomoto Co., Inc.) were mixed using a V blender (DV-1, Dalton Co., Ltd.). The resulting mixed powder was lubricated with 28 g of calcium stearate (Nitto Chemical Industry Co., Ltd.) and 14 g of light anhydrous silicic acid (Adsolider 101, Freund). The resulting lubricated powder was compression-molded with a rotary tableting machine (HT-X20SS, Hata Iron Works Co., Ltd.; tablet weight, 150.0 mg; die and punch, 10 x 5 mm; table rotation speed, 30 rpm; punch pressure, 6.9 kN)

### Example 2

| | |
|---|---|
| Mitiglinide calcium hydrate: | 10.0 mg |
| Microcrystalline cellulose: | 25.0 mg |
| Polyvinylacetal diethylaminoacetate: | 1.5 mg |
| D-mannitol: | 73.7 mg |
| Partially pregelatinized starch: | 2.4 mg |
| Corn starch: | 32.4 mg |
| Red ferric oxide: | 0.005 mg |
| Aspartame: | 2.0 mg |
| Calcium stearate: | 2.0 mg |
| Light anhydrous silicic acid: | 1.0 mg |
| Total: | 150.0 mg/tablet |

50 g of mitiglinide calcium hydrate and 125 g of microcrystalline cellulose (Ceolus PH-101, Asahi Kasei Chemicals Corporation) were mixed using a high shear granulator (FM-VG-01, Powrex Corporation). For granulation, a solution prepared by dissolving 7.5 g of polyvinylacetal diethylaminoacetate (AEA, Sankyo) in 67.5 g of 90 weight% ethanol was sprayed onto the mixture using a two-fluid spray nozzle at a feed rate of 7.5 g/min. Here, the mixture was granulated for a total of 11 minutes at a blade rotation speed of 600 rpm, and a cross screw rotation speed of 2,000 rpm. The wet granulated material was dried with a tray drier (DSB80HPT, Seiwa Rikou), and put through a sieve having a 500 µm opening. The resulting sieved granulated material containing mitiglinide calcium hydrate (a-2) had a 50% particle diameter of 119.4 µm.
0.73 g of sieved granulated material containing mitiglinide calcium hydrate (a-2), 1.57 g of the fluidized bed granulated material (b-1) prepared in Example 1, 0.6 g of corn starch, and 0.04 g of aspartame (Ajinomoto Co., Inc.) were mixed in a plastic bag. The resulting mixture was lubricated with 0.04 g of calcium stearate (Nitto Chemical Industry Co., Ltd.) and 0.02 g of light anhydrous silicic acid (Adsolider 101, Freund). The resulting lubricated powder was compression-molded with a single punch tableting machine (N-30E, Okada Seiko Co., Ltd.; tablet weight, 150.0 mg; die and punch, ∅ 7 mm; punch pressure, 6 kN).

### Example 3

| | |
|---|---|
| Mitiglinide calcium hydrate: | 10.0 mg |
| Microcrystalline cellulose: | 25.0 mg |
| Aminoalkyl methacrylate copolymer E: | 1.3 mg |
| Lactose: | 77.0 mg |
| D-mannitol: | 1.7 mg |
| Corn starch: | 30.0 mg |
| Aspartame: | 2.0 mg |
| Calcium stearate: | 2.0 mg |
| Light anhydrous silicic acid: | 1.0 mg |
| Total: | 150.0 mg/tablet |

300 g of mitiglinide calcium hydrate and 750 g of microcrystalline cellulose (Ceolus PH-101, Asahi Kasei Chemicals Corporation) were mixed using a high shear granulator (FM-VG-10, Powrex Corporation). For granulation, a solution prepared by dissolving 39 g of aminoalkyl methacrylate copolymer E (Eudragit E100, Roehm Pharma Gmbh) in 351 g of 90 weight% ethanol was sprayed onto the mixture using a two-fluid spray nozzle at a feed rate of 78 g/min. Here, the mixture was granulated for a total of 10 minutes at a blade rotation speed of 354 rpm, and a cross screw rotation speed of 2,000 rpm. The wet granulated material was dried with a tray drier (DSB80HPT, Seiwa Rikou), and sized using a mill with a screen having a 0.5 mm opening (P-02S, Dalton Co., Ltd.). The resulting sized granulated material containing mitiglinide calcium hydrate (a-3) had a 50% particle diameter of 86.3 µm.
145.2 g of sized granulated material containing mitiglinide calcium hydrate (a-3), 308 g of lactose (Tablettose 80, Meggle), 6.8 g of D-mannitol (Mannit P, Towa Kasei Kogyo), 120 g of corn starch (Nihon Shokuhin Kako Co., Ltd.), and 8 g of aspartame (Ajinomoto Co., Inc.) were mixed using a V blender (DV-1, Dalton Co., Ltd.). The resulting mixture was lubricated with 8 g of calcium stearate (Nitto Chemical Industry Co., Ltd.), and 4 g of light anhydrous silicic acid (Adsolider 101, Freund). The resulting lubricated powder was compression-molded with a rotary tableting machine (Correct12HUK, Kikusui Seisakusho Ltd.; tablet weight, 150 mg, die and punch, 10 x 5 mm; table rotation speed, 30 rpm; punch pressure, 9.8 kN).

### Example 4

| | |
|---|---|
| Mitiglinide calcium hydrate: | 10.0 mg |
| Microcrystalline cellulose: | 25.0 mg |
| Ethyl acrylate-methyl methacrylate copolymer: | 3.0 mg |
| Lactose: | 77.0 mg |
| Corn starch: | 30.0 mg |
| Aspartame: | 2.0 mg |
| Calcium stearate: | 2.0 mg |
| Light anhydrous silicic acid: | 1.0 mg |
| Total: | 150.0 mg/tablet |

50 g of mitiglinide calcium hydrate, and 125 g of microcrystalline cellulose (Ceolus PH-101, Asahi Kasei Chemicals Corporation) were mixed using a high shear granulator (FM-VG-01, Powrex Corporation). For granulation, 125 g of a 12 weight% ethyl acrylate-methyl methacrylate copolymer dispersion liquid (Eudragit NE30D, Roehm Pharma Gmbh) was sprayed onto the mixture using a two-fluid spray nozzle at a feed rate of 5 g/min. Here, the mixture was granulated for a total of 60 minutes at a blade rotation speed of 600 rpm, and a cross screw rotation speed of 2,000 rpm. The wet granulated material was dried with a fluidized bed drier (LAB-1, Powrex Corporation), and put through a sieve having a 500 µm opening. The resulting sieved granulated material containing mitiglinide calcium hydrate (a-4) had a 50% particle diameter of 77.1 µm.
152 g of sieved granulated material containing mitiglinide calcium hydrate (a-4), 308 g of lactose (Tablettose 80, Meggle), 120 g of corn starch (Nihon Shokuhin Kako Co., Ltd.), and 8 g of aspartame (Ajinomoto Co., Inc.) were mixed using a V blender (DV-1, Dalton Co., Ltd.). The resulting mixture was lubricated with 8 g of calcium stearate (Nitto Chemical Industry Co., Ltd.) and 4 g of light anhydrous silicic acid (Adsolider 101, Freund). The resulting lucricated powder was compression-molded with a rotary tableting machine (Correctl2HUK, Kikusui Seisakusho Ltd.; tablet weight, 150.0 mg; die and punch, 10 x 5 mm; table rotation speed, 30 rpm; punch pressure, 9.8 kN).

### Example 5

| | |
|---|---|
| Mitiglinide calcium hydrate: | 10.0 mg |
| Microcrystalline cellulose: | 25.0 mg |
| Ethyl cellulose: | 0.9 mg |
| D-mannitol: | 74.2 mg |
| Partially pregelatinized starch: | 2.4 mg |
| Red ferric oxide: | 0.005 mg |
| Corn starch: | 32.4 mg |
| Aspartame: | 2.0 mg |
| Calcium stearate: | 2.0 mg |
| Light anhydrous silicic acid: | 1.0 mg |
| Total: | 149.9 mg/tablet |

70 g of mitiglinide calcium hydrate and 175 g of microcrystalline cellulose (Ceolus PH-101, Asahi Kasei Chemicals Corporation) were mixed using a high shear granulator (FM-VG-01, Powrex Corporation). For granulation, a solution prepared by dissolving 6.4 g of ethyl cellulose (STD10FP, Dow Chemical Company) in 73.6 g of 99.5 weight% ethanol was sprayed onto the mixture using a two-fluid spray nozzle at a feed rate of 10 g/min. Here, the mixture was granulated for a total of 9 minutes at a blade rotation speed of 600 rpm, and a cross screw rotation speed of 2,000 rpm. The wet granulated material was dried with a fluidized bed drier (LAB-1, Powrex Corporation), and sized using a mill with a screen having a 0.5 mm opening (P-02S, Dalton Co., Ltd.). The resulting sized granulated material containing mitiglinide calcium hydrate (a-5) had a 50% particle diameter of 68.9 µm.
0.7182 g of sized granulated material containg mitiglinide calcium hydrate (a-5), 1.5818 g of fluidized bed granulated material (b-1) prepared in Example 1, 0.6 g of corn starch, and 0.04 g of aspartame (Ajinomoto Co., Inc.) were mixed in a plastic bag. The resulting mixture was lubricated with 0.04 g of calcium stearate (Nitto Chemical Industry Co., Ltd.), and 0.02 g of light anhydrous silicic acid (Adsolider 101). The resulting lubricated powder was compression-molded with a single- punch tableting machine (N-30E, Okada Seiko Co., Ltd.; tablet weight, 149.9 mg; die and punch, ∅ 7 mm; punch pressure, 5.5 kN).

**[Table 2]**

| | Example 1 | Example 2 | Example 3 | Example 4 | Example 5 |
|---|---|---|---|---|---|
| 50% particle diameter (µm) | 75.5 | 119.4 | 86.3 | 77.1 | 68.9 |
| Bitterness (score) | 1.2 | 1.2 | 1.4 | 1.6 | 1.8 |
| Oral disintegration time (sec) | 23 | 24 | 19 | 18 | 22 |
| Hardness (N) | 60 | 62 | 51 | 57 | 65 |
| Dissolution rate (%, purified water) | 92.5 | 86.3 | 90.8 | 94.7 | 93.0 |
| Dissolution rate (%, first fluid) | 97.2 | 96.2 | 97.5 | 87.0 | 92.1 |

### Comparative Example 1

| | |
|---|---|
| Mitiglinide calcium hydrate: | 10.0 mg |
| Granules of co-spray dried lactose and starch: | 135.0 mg |
| Aspartame: | 2.0 mg |
| Calcium stearate: | 2.0 mg |
| Light anhydrous silicic acid: | 1.0 mg |
| Total: | 150.0 mg/tablet |

A tablet was prepared according to the method described in Example 1 of WO2003/61650.
0.2 g of mitiglinide calcium hydrate, 2.7 g of granules of co-spray dried lactose and starch (Starlac, Meggle), and 0.04 g of aspartame (Ajinomoto Co., Inc.) were mixed in a plastic bag. The resulting mixture was lubricated with 0.04 g of calcium stearate (Nitto Chemical Industry Co., Ltd.) and 0.02 g of light anhydrous silicic acid (Adsolider 101). The resulting lubricated powder was compression-molded with a single punch tableting machine (N-30E, Okada Seiko Co., Ltd.; tablet weight, 150.0 mg; die and punch, ∅ 7 mm; punch pressure, 6 kN).

### Comparative Example 2

| | |
|---|---|
| Mitiglinide calcium hydrate: | 10.0 mg |
| Microcrystalline cellulose: | 25.0 mg |
| D-mannitol: | 75.9 mg |
| Partially pregelatinized starch: | 2.5 mg |
| Corn starch: | 32.5 mg |
| Red ferric oxide: | 0.005 mg |
| Aspartame: | 2.0 mg |
| Calcium stearate: | 2.0 mg |
| Light anhydrous silicic acid: | 1.0 mg |
| Total: | 150.9 mg/tablet |

50 g of mitiglinide calcium hydrate and 125 g of microcrystalline cellulose (Ceolus PH-101, Asahi Kasei Chemicals Corporation) were mixed using a high shear granulator (FM-VG-01, Powrex Corporation). For granulation, 140 g of ethanol was sprayed onto the mixture using a two-fluid spray nozzle at a feed rate of 9.3 g/min. Here, the mixture was granulated for a total of 18 minutes at a blade rotation speed of 600 rpm, and a cross screw rotation speed of 2,000 rpm. The wet granulated material was dried with a tray drier (DSB80HPT, Seiwa Rikou), and put through a sieve having a 500 µm opening. The resulting sieved granulated material containing mitiglinide calcium hydrate (a-6) had a 50% particle diameter of 82.6 µm.
1.4 g of sieved granulated material containing mitiglinide calcium hydrate (a-6), 3.24 g of the fluidized bed granulated material (b-1) prepared in Example 1, 1.2 g of corn starch (Nihon Shokuhin Kako Co., Ltd.), and 0.04 g of aspartame (Ajinomoto Co., Inc.) were mixed in a plastic bag. The resulting mixture was lubricated with 0.08 g of calcium stearate (Nitto Chemical Industry Co., Ltd.), and 0.04 g of light anhydrous silicic acid (Adsolider 101, Freund). The resulting lubricated powder was compression-molded with a single punch tableting machine (N-30E, Okada Seiko Co., Ltd.; tablet weight 150.9 mg; die and punch, ∅ 7 mm; punch pressure, 9.8 kN).

### Comparative Example 3

120 g of mitiglinide calcium hydrate was charged in a high shear granulator (FM-VG-01, Powrex Corporation), and a solution, prepared by dissolving 6 g of polyvinylacetal diethylaminoacetate (AEA, Sankyo) in 69 g of 90 weight% ethanol, was sprayed onto the mixture using a two-fluid spray nozzle. However, the granulation was failed because the mitiglinide calcium hydrate was not stirred in the granulator.

### Comparative Example 4

| | |
|---|---|
| Mitiglinide calcium hydrate: | 10.0 mg |
| D-mannitol: | 25.0 mg |
| Ethyl acrylate-methyl methacrylate copolymer: | 2.6 mg |
| D-mannitol: | 72.6 mg |
| Partially pregelatinized starch: | 2.4 mg |
| Red ferric oxide: | 0.005 mg |
| Corn starch: | 32.4 mg |
| Aspartame: | 2.0 mg |
| Calcium stearate: | 2.0 mg |
| Light anhydrous silicic acid: | 1.0 mg |
| Total: | 150.0 mg/tablet |

70 g of mitiglinide calcium hydrate, and 175 g of D-mannitol (Mannit P, Towa Kasei Kogyo) were mixed using a high shear granulator (FM-VG-01, Powrex Corporation). For granulation, 60 g of a 30 weight% ethyl acrylate-methyl methacrylate copolymer dispersion liquid (Eudragit NE30D, Roehm Pharma Gmbh) was sprayed onto the mixture using a two-fluid spray nozzle at a feed rate of 10.9 g/min. Here, the mixture was granulated for a total of 6.5 minutes at a blade rotation speed of 600 rpm, and a cross screw rotation speed of 2,000 rpm. The wet granulated material was dried with a fluidized bed drier (LAB-1, Powrex Corporation), and sized using a mill with a screen having a 0.5 mm opening (P-02S, Dalton Co., Ltd.). The resulting sized granulated material containing mitiglinide calcium hydrate (a-7) had a 50% particle diameter of 79.4 µm.
0.7512 g of sized granulated material containing mitiglinide calcium hydrate (a-7), 1.5488 g of the fluidized bed granulated material (b-1) prepared in Example 1, 0.6 g of corn starch, and 0.04 g of aspartame (Ajinomoto Co., Inc.) were mixed in a plastic bag. The resulting mixture was lubricated with 0.04 g of calcium stearate (Nitto Chemical Industry Co., Ltd.) and 0.02 g of light anhydrous silicic acid (Adsolider 101, Freund). The resulting lubricated powder was compression-molded with a single punch tableting machine (N-30E, Okada Seiko Co., Ltd.; tablet weight, 150.0 mg; die and punch, ∅ 7 mm; punch pressure, 5.5 kN).

### Comparative Example 5

| | |
|---|---|
| Mitiglinide calcium hydrate: | 10.0 mg |
| Microcrystalline cellulose: | 25.0 mg |
| Polyvinylacetal diethylaminoacetate: | 1.2 mg |
| D-mannitol: | 74.1 mg |
| Partially pregelatinized starch: | 2.5 mg |
| Red ferric oxide: | 0.005 mg |
| Corn starch: | 32.5 mg |
| Aspartame: | 2.0 mg |
| Calcium stearate: | 2.0 mg |
| Light anhydrous silicic acid: | 1.0 mg |
| Total: | 150.3 mg/tablet |

50 g of mitiglinide calcium hydrate, and 125 g of microcrystalline cellulose (Ceolus PH-101, Asahi Kasei Chemicals Corporation) were mixed using a high shear granulator (FM-VG-01, Powrex Corporation). For granulation, a solution prepared by dissolving 6 g of polyvinylacetal diethylaminoacetate (AEA, Sankyo) in 69 g of 90 weight% ethanol was sprayed onto the mixture using a two-fluid spray nozzle at a feed rate of 3.3 g/min. Here, the mixture was granulated for a total of 24 minutes at a blade rotation speed of 600 rpm, and a cross screw rotation speed of 2,000 rpm. The wet granulated material was dried with a tray drier (DSB80HPT, Seiwa Rikou), and put through a sieve having a 500 µm opening. The resulting sieved granulated material containing mitiglinide calcium hydrate (a-8) had a 50% particle diameter of 57.2 µm.
1.448 g of sieved granulated material containing mitiglinide calcium hydrate (a-8), 3.16 g of the fluidized bed granulated material (b-1) prepared in Example 1, 1.2 g of corn starch (Nihon Shokuhin Kako Co., Ltd.), and 0.04 g of aspartame (Ajinomoto Co., Inc.) were mixed in a plastic bag. The resulting mixture was lubricated with 0.08 g of calcium stearate (Nitto Chemical Industry Co., Ltd.), and 0.04 g of light anhydrous silicic acid (Adsolider 101, Freund). The resulting lubricated powder was compression-molded with a single punch tableting machine (N-30E, Okada Seiko Co., Ltd.; tablet weight, 150.3 mg; die and punch, ∅ 7 mm; punch pressure, 6 kN).

### Comparative Example 6

| | |
|---|---|
| Mitiglinide calcium hydrate: | 10.0 mg |
| Microcrystalline cellulose: | 25.0 mg |
| Aminoalkyl methacrylate copolymer E: | 1.3 mg |
| Lactose: | 78.7 mg |
| Corn starch: | 30.0 mg |
| Aspartame: | 2.0 mg |
| Calcium stearate: | 2.0 mg |
| Light anhydrous silicic acid: | 1.0 mg |
| Total: | 150.0 mg/tablet |

300 g of mitiglinide calcium hydrate, and 750 g of microcrystalline cellulose (Ceolus PH-101, Asahi Kasei Chemicals Corporation) were mixed using a high shear granulator (FM-VG-10, Powrex Corporation). For granulation, a solution prepared by dissolving 39 g of aminoalkyl methacrylate copolymer E (Eudragit E100, Roehm Pharma Gmbh) in 351 g of 90 weight% ethanol was sprayed onto the mixture using a two-fluid spray nozzle at a feed rate of 133.3 g/min. Here, the mixture was granulated for 3 minutes at a blade rotation speed of 600 rpm and a cross screw rotation speed of 2,000 rpm. At the same rotation speeds, the mixture was further granulated for 1 more minute while spraying 100 g of 90 weight% ethanol using a two-fluid spray nozzle at a feed rate of 100 g/min. The wet granulated material was dried with a fluidized bed drier (LAB-1, Powrex Corporation), and sized using a mill with a screen having a 0.5 mm opening (P-02S, Dalton Co., Ltd.). The resulting sized granulated material containing mitiglinide calcium hydrate (a-9) had a 50% particle diameter of 217.0 µm.
145.2 g of sized granulated material containing mitiglinide calcium hydrate (a-9), 314.8 g of lactose (Tablettose 80, Meggle), 120 g of corn starch (Nihon Shokuhin Kako Co., Ltd.), and 8 g of aspartame (Ajinomoto Co., Inc.) were mixed using a V blender (DV-1, Dalton Co., Ltd.). The resulting mixture was lubricated with 8 g of calcium stearate (Nitto Chemical Industry Co., Ltd.), and 4 g of light anhydrous silicic acid (Adsolider 101, Freund). The resulting lubricated powder was compression-molded to prepare a tablet (tablet weight, 150.0 mg; die and punch, 10 x 5 mm; table rotation speed, 30 rpm; punch pressure, 9.8 kN).

**[Table 3]**

| | Com. Example 1 | Com. Example 2 | Com. Example 4 | Com. Example 5 | Com. Example 6 |
|---|---|---|---|---|---|
| 50% particle diameter (µm) | - | 82.6 | 79.4 | 57.2 | 217.0 |
| Bitterness (score) | 2.6 | 2.6 | 1.0 | 3.0 | 1.2 |
| Dissolution rate (%,purified water) | 96.3 | 98.8 | 81.0 | 97.9 | 68.7 |
| Dissolution rate (%, first fluid) | 53.3 | 97.9 | 70.4 | 99.8 | 92.3 |

### Industrial Applicability

An orally disintegrating tablet of the present invention has an appropriate hardness and quickly disintegrates. Further, because it rapidly dissolves in the digestive tract, an orally disintegrating tablet of the present invention is useful as a therapeutic agent for type-2 diabetes mellitus.

## Claims

1. A bitterness-masked orally disintegrating tablet, comprising:
(a) mitiglinide calcium hydrate as a bitter active ingredient;
(b) microcrystalline cellulose;
(c) at least one masking agent selected from the group consisting of aminoalkyl methacrylate copolymer E, polyvinylacetal diethylaminoacetate, an ethyl acrylate-methyl methacrylate copolymer, and ethyl cellulose;
(d) a sugar or a sugar alcohol; and
(e) at least one selected from corn starch and partially pregelatinized starch.

2. A bitterness-masked orally disintegrating tablet, comprising:
a granulated material including:
(a) mitiglinide calcium hydrate as a bitter active ingredient;
(b) microcrystalline cellulose; and
(c) at least one masking agent selected from the group consisting of aminoalkyl methacrylate copolymer E, polyvinylacetal diethylaminoacetate, an ethyl acrylate-methyl methacrylate copolymer, and ethyl cellulose;
(d) a sugar or a sugar alcohol; and
(e) at least one selected from corn starch and partially pregelatinized starch.

3. The orally disintegrating tablet according to claim 1 or 2, wherein the sugar or the sugar alcohol is lactose or D-mannitol.

4. The orally disintegrating tablet according to claim 1 or 2, wherein the sugar or the sugar alcohol is D-mannitol.

5. The orally disintegrating tablet according to claim 1 or 2, wherein the masking agent is at least one selected from aminoalkyl methacrylate copolymer E and polyvinylacetal diethylaminoacetate.

6. The orally disintegrating tablet according to claim 2, wherein the granulated material is obtained by granulating a mixture of mitiglinide calcium hydrate and microcrystalline cellulose while spraying at least one masking agent selected from the group consisting of aminoalkyl methacrylate copolymer E, polyvinylacetal diethylaminoacetate, an ethyl acrylate-methyl methacrylate copolymer, and ethyl cellulose.

7. The orally disintegrating tablet according to claim 2, wherein the granulated material has an average particle diameter of 60 to 150 µm.

8. A bitterness-masking particle for orally disintegrating tablets,
the particle including:
(a) mitiglinide calcium hydrate;
(b) microcrystalline cellulose; and
(c) at least one masking agent selected from aminoalkyl methacrylate copolymer E, polyvinylacetal diethylaminoacetate, an ethyl acrylate-methyl methacrylate copolymer, and ethyl cellulose.

9. The bitterness-masking particle according to claim 8, wherein the bitterness-masking particle has an average particle diameter of 60 to 150 µm.

10. A method for preparing a bitterness-masked orally disintegrating tablet,
the method comprising the steps of:
(1) granulating a mixture of mitiglinide calcium hydrate and microcrystalline cellulose while spraying at least one masking agent selected from the group consisting of aminoalkyl methacrylate copolymer E, polyvinylacetal diethylaminoacetate, an ethyl acrylate-methyl methacrylate copolymer, and ethyl cellulose; and
(2) compression-molding the granulated material obtained in the granulating step, after mixing the granulated material with a sugar or a sugar alcohol, and at least one selected from corn starch and partially pregelatinized starch.

11. The method according to claim 10, wherein the granulation in the granulating step is performed by a high shear granulating method.
